(19) Europäisches Patentamt
European Patent Office
Office européen des brevets

(11) Veröffentlichungsnummer: **0 398 093 A2**

# (12) EUROPÄISCHE PATENTANMELDUNG

(21) Anmeldenummer: **90108479.8**

(22) Anmeldetag: **05.05.90**

(51) Int. Cl.5: **B01D 69/12, B01D 69/14, B01D 71/54**

(30) Priorität: **18.05.89 DE 3916210**

(43) Veröffentlichungstag der Anmeldung: **22.11.90 Patentblatt 90/47**

(84) Benannte Vertragsstaaten: **BE DE FR GB IT NL**

(71) Anmelder: **BAYER AG**
**D-5090 Leverkusen 1 Bayerwerk(DE)**

(72) Erfinder: **Hildenbrand, Karlheinz, Dr.**
**Gatzenstrasse 147**
**D-4150 Krefeld 1(DE)**
Erfinder: **Dhein, Rolf, Dr.**
**Deswatinesstrasse 30**
**D-4150 Krefeld 1(DE)**
Erfinder: **Meister, Willi, Dr.**
**Bahnhofstrasse 76**
**D-4047 Dormagen(DE)**
Erfinder: **Nerger, Dittmar K., Dr.**
**c/o Mobay Corporation, Mobay Road**
**Pittsburgh, PA 15205(US)**

## (54) Verbundmembranen, Verfahren zu ihrer Herstellung und ihre Verwendung.

(57) Verbundmembranen aus einer makroporösen, Füllstoff enthaltenden Membran aus mindestens zwei unverträglichen Polymeren und einer auf dieser angebrachten porenfreien Polyurethanmembran zeigen eine verbesserte Wirkung bei der Abtrennung von gegebenenfalls mit Niederalkylresten, Hydroxyl, Chlor oder Brom substituierten Benzolen aus ihren Gemischen mit aliphatischen und/oder cycloaliphatischen Kohlenwasserstoffen, Alkoholen, Ethern, Ketonen und/oder Carbonsäureestern oder aus Abwasser.

EP 0 398 093 A2

## Verbundmembranen, Verfahren zu ihrer Herstellung und ihre Verwendung

Die Erfindung betrifft neue Verbundmembranen, Verfahren zu ihrer Herstellung und ihre Verwendung zur Abtrennung von gegebenenfalls mit Niederalkylresten, Hydroxyl, Chlor oder Brom substituierten Benzolen aus ihren Gemischen mit aliphatischen und/oder cycloaliphatischen Kohlenwassertoffen, Alkoholen, Ethern, Ketonen und/oder Carbonsäureestern oder aus Abwasser.

Membranen können zur Trennung von Stoffgemischen durch Permeation eingesetzt werden. Hierbei kann beispielsweise so vorgegangen werden, daß ein Stoffgemisch in flüssiger Phase (Feed-Lösung) an eine Seite der Membran herangeführt wird und ein Stoff daraus, eine bestimmte Gruppe von Stoffen daraus oder ein Gemisch, welches an dem einen Stoff oder an der bestimmten Gruppe von Stoffen angereichert ist, an der anderen Seite der Membran ebenfalls flüssig entnommen wird (Permeation im engeren Sinne). Der durch die Membran hindurchgetretene und an der anderen Seite wieder aufgefangene Stoff oder das beschriebene Stoffgemisch wird Permeat genannt. Man kann jedoch beispielsweise auch so vorgehen, daß das Feed in flüssiger oder gasförmiger Form, bevorzugt in flüssiger Form, an die eine Seite der Membran herangebracht wird und das Permeat auf der anderen Seite in dampfförmiger Form entnommen und danach kondensiert wird (Pervaporation).

Solche Permeationsvorgänge sind wertvolle Ergänzungen zu anderen Verfahren der Stofftrennung, wie der Destillation oder der Absorption. Insbesondere bei der Trennung azeotrop siedender Stoffgemische kann die Permeation, speziell die Pervaporation, wertvolle Dienste leisten.

Es hat bisher viele Versuche gegeben, Membranen aus verschiedenen Polymermaterialien einzelnen speziellen Zwecken anzupassen. So ist aus US 2.953.520 bekannt, aus einem azeotropen Benzol/Methanol-Gemisch mit Hilfe einer nicht porösen plastischen Membran aus Polyethylen Benzol im Permeat anzureichern und dadurch weitgehend abzutrennen. Ferner ist aus US 3.776.970 bekannt, die beiden aromatischen Verbindungen Styrol und Ethylbenzol mit Hilfe einer Membran aus bestimmten Polyurethanelastomeren so zu trennen, daß Styrol im Permeat angereichert wird. Aus DE-PS 26 27 629 ist ferner bekannt, mit Hilfe von Polyurethanmembranen Benzol und Alkylbenzole von aliphatischen Kohlenwasserstoffen, cycloaliphatischen Kohlenwasserstoffen, Alkoholen, Ethern und Carbonsäureestern abzutrennen.

Es wurde nun überraschend gefunden, daß man mit der im folgenden beschriebenen Verbundmembran die Abtrennung von gegebenenfalls mit Niederalkylresten, Hydroxyl, Chlor oder Brom substituierten Benzolen aus ihren Gemischen mit aliphatischen und/oder cycloaliphatischen Kohlenwasserstoffen, Alkohlen, Ethern, Ketonen und/oder Carbonsäureestern oder aus Abwasser gegenüber den in DE-PS 26 27 629 beschriebenen Polyurethanmembranen wesentlich verbessern kann, wobei diese verbesserten Trenneffekte insbesondere im Bereich von Gemischen mit niedrigem Aromatengehalt deutlich werden.

Die Erfindung betrifft daher Verbundmembranen, bestehend aus

i) einer makroporösen, mindestens einen Füllstoff enthaltenden Membran aus mindestens zwei unverträglichen Polymeren, wobei der Füllstoff oder ein Gemisch mehrerer von ihnen 30 - 85 % des Gesamtgewichts von Füllstoff(en) und unverträglichen Polymeren ausmacht, und

ii) einer auf i) angebrachten porenfreien Polyurethan(PU)membran.

Die makroporöse Membran nach i) besteht aus mindestens zwei Polymeren, die in Lösung unverträglich sind, d.h., in einer gemeinsamen Lösung zur Phasentrennung führen. Näheres über entmischende, unverträgliche Polymersysteme ist der Monographie Paul J. Flory, Principles of Polymer Chemistry, Ithaca, N.Y., (1953), zu entnehmen. Durch Eindispergieren mindestens eines unlöslichen Füllstoffes in diese instabile Mischung wird diese in eine stabile homogene Dispersion übergeführt. Diese Dispersion wird dann als Gießlösung auf eine Unterlage aufgetragen. Durch eine Fällungskoagulation, auch Phaseninversion genannt, wird aus dieser Gießlösung die makroporöse, Füllstoff(e) enthaltende Membran nach i) hergestellt. Diese Technologie der Phaseninversion ist bekannt, beispielsweise aus H. Strathmann, Trennungen von molekularen Mischungen mit Hilfe synthetischer Membranen, Steinkopf-Verlag, Darmstadt (1979) und D. R. Lloyds, Materials Science of Synthetic Membranes, ACS Symp. Ser. 269, Washington D.C. (1985).

In diesen Schriften sind auch die typischen Membranstrukturen beschrieben, die bei der Fällungskoagulation erhalten werden. Es handelt sich stets um asymmetrische Membranstrukturen mit einer dichteren Polymerhaut an der Membranoberfläche und höheren Porositäten im Membraninneren. Die Rorenstruktur kann je nach Rezeptur der Gießlösung fingerartig oder schaumartig sein. Durch die Ausbildung der dichteren Polymerhaut an der Membranoberfläche sind die Porendurchmesser der konventionellen Membranen beschränkt und überschreiten in der Regel Werte von etwa 8-10 µm nicht.

Bei der Herstellung von Fällungskoagulationsmembranen konventioneller Art geht man von homogenen Polymergießlösungen aus, da ansonsten instabile Membranen erhalten wurden. Aus diesem Grund entstehen typische Membrangießlösungen aus einem Polymer und einem Lösungsmittel bzw. Lösungsmittelgemisch (beispielsweise Polyamid in Dimethylacetamid oder Celluloseacetat in Aceton/Formamid).

Es hat schon Versuche gegeben, durch spezielle Rezepturen der Polymergießlösungen Membranen mit erhöhten Permeabilitäten herzustellen. So sind in Chem. Prof. Res. Dev. 22 (1983), 320-326 oder in DE-OS 31 49 976 Membranen beschrieben, zu deren Herstellung Polymergießlösungen eingesetzt wurden, die wasserlösliche Polymere wie Polyvinylpyrrolidon enthalten, die bei der Koagulation in Wasser herausgelöst werden und dadurch zu vergrößerten Poren führen. Es sind auch Membranen aus Polymergemischen beschrieben worden. Die Rezepturen der entsprechenden Gießlösungen sind jedoch so aufgebaut, daß aufgrund der Löslichkeitsparameter homogene Polymerlösungen erhalten werden. Beispielsweise werden in EP 66 408 Membranen aus einem Gemisch von Celluloseacetat und Polymethylmethacrylat beschrieben, die gegenüber den konventionellen Membranen aus nur einem Polymer erhöhte Permeabilitäten aufweisen. Man ist hierbei jedoch auf Polymerkombinationen mit ähnlichen Löslichkeitsparametern sowie auf bestimmte, sehr enge Mischungsverhältnisse angewiesen.

Überraschenderweise wurde nun gefunden, daß makroporöse Membranen aus an sich unverträglichen und nicht mischbaren Polymeren in jedem beliebigen Mischungsverhältnis zu homogenen Gießlösungen verarbeitet werden können, wenn bestimmte unlösliche Füllstoffe eindispergiert werden, und im Verbund mit darauf angebrachten porenfreien Polyurethan(PU)membranen die eingangs erwähnten besseren Trenneffekte aufzeigen.

Werden beispielsweise eine 20 gew.-%ige Lösung von Polyurethan in Dimethylformamid (PU/DMF-Lösung) und eine 20 gew.-%ige Lösung von Polyacrylnitril in Dimethylformamid (PAN/DMF-Lösung) unter Rühren vermischt, so kommt es nach kurzem Stehen zur Phasenseparation. Derartige Gemische sind instabil und als Gießlösungen für die Membranherstellung nicht geeignet. Vereinigt man dagegen dieselben Polymer/DMF-Lösungen unter gleichzeitigem oder nachträglichem Eindispergieren von Füllstoffen, beispielsweise von Talkum, so erhält man homogene, stabile Gießlösungen, die für die Membranherstellung nach der Methode der Fällungskoagulation geeignet sind.

Die aus derartigen Gießlösungen hergestellten Membranen zeigen im Vergleich zu den bekannten deutlich größere Poren an der Oberfläche und eine sehr viel höhere Gesamtporosität.

Wie die elektronenmikroskopischen Aufnahmen vom Querschnitt dieser Polymermembranen zeigen, handelt es sich um Strukturen mit einem filzartigen Aufbau, während der bekannte asymmetrische Strukturaufbau mit einer dichteren Polymerhaut an der Membranoberfläche fast vollständig zurückgedrängt ist. An der Membranoberfläche sind bei einer Membran der obigen Rezeptur durchschnittliche Porendurchmesser von bis zu 30 $\mu$m zu erkennen.

Die zur Herstellung derartiger Membranmatrizen erforderlichen Polymergießlösungen müssen die folgenden Bedingungen erfüllen:

a) Die Lösungen der einzelnen Polymerkomponenten dürfen nicht miteinander mischbar sein. Bei mischbaren Systemen werden in Analogie zu konventionellen Gießlösungen feinporöse Membranstrukturen mit ausgeprägter asymmetrischer Struktur erhalten.

b) Die Lösungsmittel der einzelnen Polymerkomponenten müssen miteinander mischbar sein.

c) Um die nicht mischbaren Polymerkomponenten in homogene Gießlösungen zu überführen, müssen geeignete unlösliche, beispielsweise anorganische Füllstoffe in einer Menge eindispergiert werden, die 30 - 85 % des Gesamtgewichts von Füllstoff(en) und unverträglichen Polymeren ausmachen. In bevorzugter Weise macht der Füllstoff bzw. ein Gemisch mehrerer von ihnen 50 - 75 % des Gesamtgewichts aus.

Die Art des Füllstoffes kann in manchen Fällen wichtig sein für die Stabilität und Homogenität der Gießlösung. Während beispielsweise Gießlösungen aus PU/PAN-Gemischen mit Titandioxid ($TiO_2$ RKB2®, Bayer AG) oder Bariumsulfat (Blanc Fixe Mikron®, Sachtleben) mit spezifischen Oberflächen von etwa 3 $m^2/g$ bezüglich der Stabilität und Homogenität weniger günstig sind, zeigen Lösungen aus dem selben Polymergemisch mit Talkum (Talkum AT 1, Norwegian Talc) eine gute Homogenität und Dispersionsstabilität.

Ähnlich gute Ergebnisse konnten auch mit sehr feinkörnigen Füllstoffen mit großer spezifischer Oberfläche er halten werden, beispielsweise mit Titandioxid Degussa P25 (etwa 40 $m^2/g$) oder Siliciumdioxid Aerosil 200® Degussa (200 $m^2/g$). Auch Gemische von Talkum mit Bariumsulfat oder Talkum mit $TiO_2$ RKB2® bzw. Titandioxid P25®, Degussa, mit Bariumsulfat führen zu geeigneten Gießlösungen. Auch durch Eindispergieren von mikrokristalliner Cellulose (beispielsweise Arbocel B E 600/30 ®, J. Rettenmaier & Söhne) konnten geeignete Gießlösungen hergestellt werden. Weitere geeignete Füllstoffe sind $CaCO_3$, $MgCO_3$, ZnO sowie Eisenoxide.

Neben den bereits genannten Füllstoffen seien noch Zeolithe und Bentonite genannt, ferner Gemische von $TiO_2$ mit $BaSO_4$ oder Talkum mit $BaSO_4$, ferner Gemische von $TiO_2$ mit großer und kleiner spezifischer Oberfläche wie $TiO_2$ RKB2® Bayer/$TiO_2$ P 25® Degussa. Bevorzugte Füllstoffe sind: Talkum, mikrokristalli-

ne Cellulose, Zeolithe, Bentonite, $BaSO_4$, $TiO_2$ und $SiO_2$.

Die Funktion und Wirkung des Füllstoffs ist die Überführung der instabilen inhomogenen Polymerlösung in stabile und homogene Gießlösungen; der Mechanismus dieser "Lösungsvermittlung" ist nicht bekannt. Durch orientierende Vorversuche können geeignet Füllstoff/Polymer-Kombinationen ermittelt werden.

Die Porengröße wird über die Auswahl der Polymere und der jeweiligen Mengenanteile gesteuert. Die Füllstoffe haben keinen oder nur einen untergeordneten Einfluß auf die Porengröße. Die Partikeldurchmesser der Füllstoffe liegen in einer kleineren Größenordnung, nämlich von 0,007 - 16 µm, vielfach 0,3 - 5 µm als die Porendurchmesser der Polymermembran (≦ 30 µm). Für die Porenbildung der erfindungsgemäßen Membranen ist der Prozeß der Fällungskoagulation in Kombination mit dem hier beschriebenen Typ von Gießlösungen verantwortlich. Der Bereich der durchschnittlichen Porengröße der erfindungsgemäßen, makroporösen Membranen beträgt 10 bis 30 µm, bevorzugt 15 bis 25 µm. Eine solche durchschnittliche Porengröße schließt das Auftreten von Poren in einem darunter liegenden Bereich (beispielsweise ab 1 µm) sowie in einem darüberliegenden Bereich (beispielsweise bis 50 µm) nicht aus.

Zur Herstellung der makroporösen, Füllstoff enthaltenden Membran nach i) können beispielsweise die folgenden Polymerklassen eingesetzt werden: Celluloseester, Polyvinylester, Polyurethane, Polyacrylderivate bzw. Acrylcopolymere, Polycarbonate und ihre Copolymere, Polysulfone, Polyamide, Polyimide, Polyhydantoine, Polystyrol bzw. Styrolcopolymere, Poly(para-dimethyl-phenylenoxid), Polyvinylidenfluorid, Polyacrylnitril, Ethylen-vinylacetat-copolymere mit mindestens 50 Gew.-% Vinylacetat.

In bevorzugter Weise werden zwei oder drei unverträgliche Polymere aus der Klasse Polyurethane, Polyacrylnitril, Polyvinylacetat, Polystyrol, Polysulfon, Polyvinylidenfluorid, Polyamid, Polyhydantoin und Ethylenvinylacetat-Copolymere mit mindestens 50 Gew.-% Vinylacetat eingesetzt. Beispiele für binäre unverträgliche Polymersysteme sind:

- Celluloseester/Polyvinylester (wie Celluloseacetat Cellidor CP®/Polyvinylacetat Mowilith®)
- Polyurethan/Polyacrylderivate (wie Desmoderm KBH®/Polyacrylnitril Dralon T® oder Desmoderm KBH®/aminmodifiziertes Dralon A® oder Desmoderm KBH®/anionisch modifiziertes, d.h. mit Sulfongruppen versehenes Dralon U®)
- Polycarbonat-Copolymere/Polyurethan (wie Polyetherpolycarbonat/Desmoderm KBH®)
- Polyvinylderivate/Polysulfone (wie Polyvinylidenfluorid/Polysulfon Udel P 1700®)
- Polyamide bzw. Polyimide/Polystyrol bzw. Styrolcopolymere
- Poly(para-dimethyl-phenylenoxid)/Polyvinylidenfluorid
- Polyhydantoin/Polystyrol

Als weitere Zweierkombinationen seien genannt:
Dralon U®/Mowilith®, Cellidor CP®/Dralon U®; ternäre Polymergemische sind beispielsweise Cellidor CP®/Dralon U®/Polystyrol, Mowilith R®/Desmoderm KBH®/Polyvinylchlorid, Desmoderm KBH®/Mowilith R®/Dralon T®, wobei Dralon T® auch durch Dralon A® ersetzt sein kann.

Bevorzugte binäre und ternäre Polymersysteme sind:
Desmoderm KBH®/Dralon T®, Desmoderm KBH®/Dralon A®, Desmoderm KBH®/Mowilith®/Dralon T®, wobei Dralon T® auch durch Dralon A® oder Dralon U® ersetzt sein kann.

Die chemischen Strukturen der bevorzugt eingesetzten Polymere sind im Anhang zu den Ausführungsbeispielen beschrieben.

Im Prinzip können auch 4 oder mehr unverträgliche Polymere eingesetzt werden, jedoch ergibt dies bei erhöhtem Aufwand keinen zusätzlichen Vorteil.

Das für die Porendurchmesser erforderliche Mengenverhältnis der Polymere in den jeweiligen Kombinationen läßt sich durch entsprechende Versuche ermitteln. Wenn die mindestens zwei Polymere in annähernd gleichen Mengen gemischt werden, erhält man in der Regel höhere Werte der durchschnittlichen Porengrößen; bei stärker unterschiedlichen Mengen erhält man niedrigere Werte. In der Polymergießlösung aus zwei Polymeren sollten mindestens 10 Gew.-% eines Polymeren, bezogen auf die Gesamtmenge aller Polymeren, vorliegen. Bei mehr als zwei unverträglichen Polymeren sollte diese Mindestmenge 8 Gew.-% eines Polymeren betragen.

Die makroporäse, Füllstoffe enthaltende Membran i) als Teil der erfindungsgemäßen Verbundmembran hat eine Dicke von 10 - 200 µm, bevorzugt 30 - 100 µm.

Zur Herstellung von Gießlösungen der bevorzugten Polymerkombinationen ist Dimethylformamid (DMF) als Lösungsmittel gut geeignet. Weitere geeignete Lösungsmittel sind in Abhängigkeit von den verwendeten Polymeren: N-Methyl-pyrrolidon (NMP), Dimethylsulfoxid (DMSO), Di methylacetamid, Dioxolan, Dioxan, Aceton, Methylethylketon oder Cellosolve®.

Die Lösungsmittelmenge wird so bemessen, daß eine Viskosität der Gießlösung erreicht wird, die im Bereich von 500 bis 25 000 mPas erreicht wird. In der Regel entspricht das einem Polymergehalt von 10 bis 40 Gew.-% an der gesamten füllstoffhaltigen Gießlösung.

Der Gesamtprozeß der Herstellung des Anteils i) an den erfindungsgemäßen Verbundmembranen läßt sich anhand eines bevorzugten Beispiels wie folgt beschreiben: Die jeweils ca. 20 gew.-%igen DMF-Polymerlösungen aus Desmoderm KBH®, Mowilith® und Dralon T® wurden unter Eindispergieren von Talkum mit Hilfe eines schnell drehenden Rührers (Dissolver) zu einer homogenen Polymergießlösung vermischt. Nach dem Entgasen im Vakuum wurde diese Gießlösung mit Hilfe eines Rakels in einer Schichtdicke von beispielsweise 150 µm auf ein Trägersubstrat aufgebracht und in das Koagulationsbad, beispielsweise reines Wasser, getaucht. Nach einer Verweilzeit von etwa 2 Min. wurde die dabei entstandene mikroporöse, Füllstoff enthaltende Membran aus dem Koagulationsbad genommen und mit Warmluft getrocknet.

Für die Herstellung der Gießlösung können auch Tenside, beispielsweise Dioctylnatriumsulfosuccinat oder Dodecylbenzolsulfonate in einer Menge von 2 - 10 % des Gesamtgewichts der Gießlösung eingesetzt werden. Auch wasserlösliche Polymere, wie Celluloseether, Polyethylenglykole, Polyvinylalkohol oder Polyvinylpyrrolidon können Bestandteil der Polymergießlösung sein. Als weitere Zusätze kommen sogenannte Koagulationshilfsmittel, wie beispielsweise kationische Polyurethandispersionen (wie Desmoderm Koagulant KPK®) in Frage. Die wasserlöslichen Polymere und die weiteren Zusätze machen 0 - 10 % des Gesamtgewichts der Gießlösung aus.

Das zum Aufbringen der Gießlösung eingesetzte Trägersubstrat kann ein solches sein, das lediglich zur Herstellung der makroporösen, Füllstoff enthaltenden Membran nach i) dient und daher nach dem Koagulationsvorgang von i) wieder abgezogen wird. Hierzu muß das Trägersubstrat glatt sein und ist beispielsweise Glas, Polyethylenterephthalatfolie oder ein silikonisiertes Trägermaterial. Soll jedoch die erfindungsgemäße Verbundmembran aus i) und ii) zur Verbesserung der mechanischen Stabilität mit einem Stützmaterial versehen sein, verwendet man als Trägersubstrat flüssigkeitsdurchlässige Materialien, wie Polymergewebe oder Polymervliese, auf denen die makroporöse, Füllstoff enthaltende Membran i) eine gute Haftung zeigt. Die Mitbenutzung eines solchen Stützmaterials (Gewebe oder Vlies) ist für die erfindungsgemäßen Verbundmembranen bevorzugt. Geeignete Materialien hierzu sind: Polypropylen- oder Polyestervliese, multifile Polyester-, multifile Polyamid- oder multifile Glasfasergewebe.

Es ist ferner bekannt, zur Vergrößerung der Oberfläche von Membranen diese außer in der Form von Folien, deren Herstellung soeben beschrieben wurde, auch in Form von Röhren, Schläuchen oder Hohlfasern zu verwenden. Diese können zur Erreichung von maximalen Membranoberflächen bei möglichst geringen Apparatevolumina in speziellen Trenneinheiten angeordnet und verwendet werden, die Module genannt werden. Solche Röhren, Schläuche oder Hohlfasern können beispielsweise dadurch hergestellt werden, daß bei einer konzentrischen Zweistoffdüse durch den äußeren Ringspalt die oben beschriebene, Füllstoff enthaltende und dadurch stabilisierte Gießlösung gepreßt wird, während durch die zentrale Düsenöffnung ein Koagulationsmittel wie Wasser gepreßt wird und die austretende Gießlösung außerdem in ein Koagulationsbad wie Wasser eintritt; dadurch wird die Koagulation von innen und von außen vorgenommen.

Auf die makroporöse, Füllstoff enthaltende Membran i) wird nach Koagulation und Trocknen eine porenfreie Polyurethan(PU)membran durch Gießtechnik aufgebracht.

Die Stärke dieser porenfreien PU-Membran beträgt 0,5-500 µm, bevorzugt 5-50 µm.

Polyurethane für diese porenfreie PU-Membran ii) und ihre Herstellung sind bekannt. Polyurethane werden im allgemeinen durch Umsetzung von höhermolekularen Di-oder Polyhydroxyverbindungen und aliphatischen, araliphatischen oder aromatischen Di- oder Polyisocyanaten sowie gegebenenfalls sogenannten Kettenverlängerungsmitteln hergestellt.

Als OH-Endgruppen enthaltende Ausgangsmaterialien seien beispielsweise genannt: Polyester der Kohlensäure und aliphatischer Dicarbonsäuren mit 2-10 C-Atomen, bevorzugt der Adipin- und Sebacinsäure, mit aliphatischen Dialkoholen mit 2-10 C-Atomen, bevorzugt solchen mit 2 bis 6 C-Atomen, wobei die Dialkohole auch zur Erniedrigung der Schmelzpunkte der Polyester im Gemisch eingesetzt werden können; Polyester aus niedermolekularen aliphatischen Lactonen und ω-Hydroxycarbonsäuren, bevorzugt aus Caprolacton bzw. ω-Hydroxycaprinsäure, deren Carboxylgruppen mit Diolen umgesetzt worden sind; ferner Polyalkylenetherdiole, speziell Polytetramethylenetherdiole, Polytrimethylenetherdiole, Polypropylenglykol oder entsprechende Copolyether.

Als Diisocyanate werden aromatische Diisocyanate, wie Toluylendiisocyanat, m-Xylylendiisocyanat, araliphatische Diisocyanate, wie Diphenylmethan-4,4′-diisocyanat oder auch aliphatische und cycloaliphatische Diisocyanate, wie Hexamethylendiisocyanat und Dicyclohexylmethan-4,4′-diisocyanat sowie Isophorondiisocyanat verwendet.

Diese Ausgangsmaterialien können gegebenenfalls mit zusätzlich eingesetzten Dialkoholen auch zu sogenannten Prepolymeren umgesetzt und anschließend mit weiteren Di- oder Polyhydroxylverbindungen und Di- oder Polyisocyanaten und gegebenenfalls weiteren Kettenverlängerungsmittel erneut polymerisiert

werden. Neben den bei Verwendung von Diolen und Diisocyanaten erhältlichen zweidimensional vernetzten Polyurethanen können auch dreidimensional vernetzte Polyurethane erhalten werden, wenn bei der Polymerisation gleichzeitig Trihydroxyverbindungen und/oder Polyole und/oder Tris- und/oder Polyisocyanate als Ausgangsmaterialien eingesetzt werden.

Eine dreidimensionale Vernetzung läßt sich jedoch auch erreichen, wenn man noch freie Hydroxyl- und/oder Isocyanat-Gruppen enthaltende zweidimensional vernetzte Polyurethane anschließend mit mehrfunktionellen Alkoholen und/oder Isocyanaten weiter umsetzt. Gleichfalls lassen sich solche dreidimensional vernetzten Polyurethane durch anschließende Umsetzung von zweidimensional vernetzten Polyurethanen mit freien Isocyanat-Endgruppen mit geringen Mengen von Polymeren mit reaktionsfähigen Wasserstoffatomen enthaltenden Endgruppen wie Formaldehyd- oder Melamin-Harzen erhalten. Für die porenfreien PU-Membranen ii) werden bevorzugt filmbildende elastische Polyurethane verwendet, die als sogenannte "Einkomponenten-PU" mit einer Kennzahl (Äquivalent)

$$\frac{NCO}{OH} \quad \text{bzw.} \quad \frac{NCO}{OH + NH_2}$$

von etwa 1,0, etwa im Bereich 0,95 bis 1,1, hergestellt werden. Dabei werden als Diole insbesondere Butandiol-1,4-adipinsäurepolyester, Hexamethylenglykol-1,6-adipinsäurepolyester und Hexandiol-1,6-polycarbonat eingesetzt.

Als Diisocyanate kommen bevorzugt Isophoron-diisocyanat, 4,4′-Diisocyanato-diphenylmethan und Toluylen-diisocyanat in Frage. Als Kettenverlängerungsmittel werden bevorzugt Ethylenglykol, Butandiol-1,4, Ethanolamin und Diamino-dicyclohexyl-methan verwendet.

Zu dieser Gruppe gehören auch Polyurethane, die aus einem Prepolymeren mit freien Hydroxylgruppen, einem Diol und Diisocyanat mit einer Kennzahl

$$\frac{NCO}{OH}$$

etwa gleich 1
hergestellt sind.

Eine weitere bevorzugte Gruppe derartiger filmbildender Polyurethane sind sogenannte "Zweikomponenten-PU" eines der vorgenannten Polyurethane, die durch anschließende weitere Polymerisation mit einem Polyol wie Trimethylolpropan und gegebenenfalls einem Kettenverlängerer wie 1,3-Butylenglykol und einem Diisocyanat vernetzt worden sind. Zu dieser Gruppe der "Zweikomponenten-PU" gehören auch solche Polyurethane, die anschließend mit Formaldehyd- oder Melamin-Harzen weiter vernetzt wurden.

Selbstverständlich können für die Herstellung der porenfreien PU-Membran ii), wie sie in den erfindungsgemäßen Verbundmembranen eingesetzt werden, auch andere Polyurethane eingesetzt werden; lediglich solche Polyurethane sind nicht geeignet, die sich in den zu trennenden aromatischen und aliphatischen bzw. cycloaliphatischen Kohlenwasserstoffen auflösen.

Neben der obenerwähnten Gießtechnik zum Aufbringen der porenfreien PU-Membran ii) auf die mikroporöse, Füllstoff enthaltende Membran i) ist grundsätzlich auch das Aufbringen durch Extrusion, Kalandrieren oder Spritztechnik denkbar. Das Aufbringen durch Gießtechnik ist jedoch bevorzugt.

Innerhalb der Gießtechnik ist es eine mögliche Ausführungsform, der PU-Gießlösung Acrylate zuzusetzen. Diese zugesetzten Acrylate ermöglichen es, die porenfreie PU-Membran ii) innerhalb der erfindungsgemäßen Verbundmembranen durch UV-Bestrahlung oder $\gamma$-Strahlung oder Elektronenstrahlen zu vernetzen und dadurch mechanisch zu stabilisieren.

Als Acrylate kommen Acrylsäure- und/oder Methacrylsäureester von Diolen mit 4-12 C-Atomen oder von Tri- bzw. Tetraalkoholen in Frage, insbesondere Butandiol-1,4-acrylat, Butandiol-bis-methacrylat, besonders aber Trimethylolpropan-trisacrylat, Trimethylolpropan-trimethacrylat oder Pentaerithrit-tetraacrylat bzw. Pentaerithrit-tetramethacrylat oder Urethanacrylate (bei spielsweise Umsetzungsprodukte aus Trimethylolpropan, Isophorondiisocyanat und Hydroxyethylacrylat) in Frage. Ihre Menge beträgt 4-24 Gew.-%, bezogen auf die Gesamtmenge an Polyurethan und Acrylaten. Man erhält damit ein vernetzbares Acrylat-Polyurethan-Blend für ii). In besonders bevorzugter Weise wird Trimethylpropan-trisacrylat eingesetzt.

Geht man zur Herstellung der porenfreien PU-Membran ii) von wäßrigen PU-Dispersionen (Angew. Makromolek. Chemie 98 (1981) 133-165) aus, können diese zur Verbesserung der mechanischen Festigkeit gegebenenfalls mit Carbodiimiden vernetzt werden.

Ferner können bei der Herstellung der PU-Membran ii) auch Weichmacher wie Nonylphenol oder

Füllstoffe wie feinteiliges $SiO_2$ (beispielsweise Kieselgel oder Aerosiol-Typen der Fa. Degussa) sowie Zeolithe eingesetzt werden.

Die Erfindung betrifft weiterhin die Herstellung von Verbundmembranen der obengenannten Art, die dadurch gekennzeichnet ist, daß man

a) in eine Lösung, enthaltend wenigstens zwei unverträgliche Polymere in Mengen, die zu einer Phasentrennung in der Lösung führen, mindestens einen unlöslichen Füllstoff eindispergiert, wobei der Füllstoff oder ein Gemisch mehrerer von ihnen 30 - 85 % des Gesamtgewichts von Füllstoff(en) und unverträglichen Polymeren ausmacht und wobei eine homogene Gießlösung entsteht,

b) diese Lösung zu Membranen in Form von Folien, Röhren, Schläuchen oder Hohlfasern verarbeitet und eine Fällungskoagulation durchführt und

c) auf die hierbei erhaltene makroporöse, Füllstoff enthaltende Membran eine porenfreie PU-Membran aufbringt.

Bei der Herstellung der Membranen im Schritt b) in Form von Folien wird die Lösung auf ein Trägersubstrat aufgebracht, und nach der Fällungskoagulation in der oben beschriebenen Weise vor der Durchführung des Schrittes c) wird das Koagulat vom Trägersubstrat abgelöst.

In bevorzugter Weise wird dieses Verfahren jedoch so modifiziert, daß das Trägersubstrat ein Stützmaterial der genannten Art ist, welches an der Verbundmembran verbleibt. Anschließend wird im Gießverfahren die porenfreie PU-Membran ii) in der oben beschriebenen Weise aufgebracht.

Für den Fall der Herstellung der erfindungsgemäßen Verbundmembranen in der Form von Rohren, Schläuchen oder Hohlfasern wird nach der Herstellung der makroporösen, Füllstoffe enthaltenden Membran i), beispielsweise durch Extrusion und Koagulation in der oben beschriebenen Weise, eine PU-Gießlösung zur Herstellung der porenfreien PU-Membran ii) durch Gießen an das Innere solcher Röhren, Schläuche oder Hohlfasern gebracht, wobei gegebenenfalls mit einem Inertgas nachgespült wird, um bei spielsweise bei Hohlfasern ein Verkleben des Inneren zu vermeiden. Dieses Inertgas kann gleichzeitig vorgewärmt sein, um das Verdunsten des Lösungsmittels aus der Gießlösung zu bewirken. Eine solche Art der Anbringung von ii) ist geeignet, um das aufzutrennende Gemisch aus gegebenenfalls mit Niederalkylresten, Hydroxyl, Chlor oder Brom substituierten Benzolen und aliphatischen und/oder cycloaliphatischen Kohlenwasserstoffen, Alkoholen, Ethern, Ketonen und/oder Carbonsäureestern oder das solche Benzole enthaltende Abwasser in das Innere dieser Röhren, Schläuche oder Hohlfasern zu bringen und das am gegebenenfalls substituierten Benzol angereicherte Permeat an der äußeren Oberfläche der Röhren, Schläuche oder Hohlfasern abnehmen. Diese Art des Aufbaus der erfindungsgemäßen Verbundmembranen ist dann besonders günstig, wenn von der Mischungsseite zur Permeatseite ein Druckgefälle von einem höheren zu einem tieferen Druck angebracht werden soll.

Daneben ist grundsätzlich auch der umgekehrte Einsatz möglich, nämlich das Heranbringen des Ausgangsgemisches an die äußere Oberfläche der Röhren, Schläuche oder Hohlfasern und der Abzug des Permeats von der inneren Oberfläche. Für diese Ausführungsform muß die PU-Gießlösung zur Herstellung von ii) an die äußere Oberfläche von Röhren, Schläuchen oder Hohlfasern aus der makroporösen, Füllstoff enthaltenden Membran i) herangebracht werden.

Die Erfindung betrifft weiterhin die Verwendung der oben beschriebenen Verbundmembranen für die Abtrennung von Benzol, das ein- bis dreifach durch Chlor, Brom, $C_1$-$C_4$-Alkyl oder Hydroxyl substituiert sein kann, aus aliphatischen und/ oder cycloaliphatischen Kohlenwasserstoffen, Alkoholen, Ethern, Ketanen und/oder Carbonsäureester oder aus Abwasser.

Gegebenenfalls substituierte Benzole sind: Benzol, Toluol, Xylol, Ethylbenzol, Propylbenzol, Chlorbenzol, Dichlorbenzol, Brombenzol, Phenol oder Kresol.

Aliphatische bzw. cycloaliphatische Kohlenwasserstoffe, aus denen das gegebenenfalls substituierte Benzol abgetrennt werden soll, sind beispielsweise geradkettige oder verzweigte Kohlenwasserstoffe mit 5-14 C-Atomen, wie Pentan, Hexan, Heptan, 2-Methyl- und 3-Methylhexan, 2,2-Dimethylpentan, 2,4-Dimethylpentan, 2,2,3-Trimethylbutan, geradkettiges oder verzweigtes Tetradecan, i-Octan oder cycloaliphatische Kohlenwasserstoffe, insbesondere mit 5 und 6 Ring-C-Atomen, die auch durch $C_1$-$C_8$-Alkyl, bevorzugt $C_1$-$C_4$-Alkyl, besonders bevorzugt durch Methyl und Ethyl substituiert sein können. Diese aliphatischen bzw. cycloaliphatischer Kohlenwasserstoffe können einzeln oder als Gemisch vorliegen; bevorzugte Gemische sind solche petrochemischer Herkunft, beispielsweise für Kraftstoffe geeignete. Bevorzugte cycloaliphatische Kohlenwasserstoff hierin sind Methylcyclopentan, Cyclohexan und Methylcyclohexan. Ebenso können auch mehr als ein gegebenenfalls substituiertes Benzol zur Abtrennung im Gemisch vorhanden sein.

Als weitere organische Lösemittel, aus denen sich gegebenenfalls substiutierte Benzole mit Hilfe der erfindungsgemäßen Membran abtrennen lassen, kommen Alkohole, wie Ethanol; Ether, wie Dioxan; Ketone, wie Cyclohexanon und Carbonsäureester, wie Ethylacetat in Frage.

Die Abtrennung erfolgt durch Flüssig-Flüssig-Permeation, Gasförmig-Gasförmig-Pervaporation oder

Flüssig-Gasförmig-Pervaporation, bevorzugt durch Flüssig-Gasförmig-Pervaporation. Die hierzu erforderlichen Techniken sind dem Fachmann bekannt. In bevorzugter Weise wird mit einem Druckgefälle in Richtung des Permeats gearbeitet, wozu auf der Permeatseite ein Unterdruck (z.B. 1-500 mbar) angelegt wird.

Es ist überraschend, daß die erfindungsgemäßen Verbundmembranen einen deutlichen verbesserten Trennfaktor für gegebenenfalls substituierte Benzole besitzen.

Als Maß für die Abtrennwirkung wird allgemein der Trennfaktor α angegeben, der ein Maß für die selektive Permeabilität der Membran darstellt; er ist durch folgende Gleichung definiert:

$$\alpha = \frac{C_{Ap}}{C_{Bp}} \times \frac{C_{Bg}}{C_{Ag}} ,$$

in der

$C_{Ap}$ und $C_{Bp}$ die Konzentrationen der Stoffe A und B im Permeat (p), sowie $C_{Ag}$ und $C_{Bg}$ die entsprechenden Konzentrationen im zu trennenden Gemisch (g) bedeuten, worin A jeweils die abzutrennende Komponente, im vorliegenden Fall das gegebenenfalls substituierte Benzol (oder mehrere Benzole), und B die andere oder die übrigen Komponenten des Gemisches bedeuten.

Ein sehr überraschender Effekt der erfindungsgemäßen Verbundmembranen ist ihr erfolgreicher Einsatz zur Abtrennung von gegebenenfalls substituiertem Benzol aus Abwasser.

## Beispiel 1

a) Herstellung der makroporösen, Füllstoffe enthaltenden Polymerblendmembran:

21,6 g einer 17 %igen Dralon T®/DMF-Lösung,
65,2 g einer 20 %igen KBH®-Polyurethan/DMF-Lösung,
86,6 g einer 25 %igen Mowilith 50®/DMF-Lösung,
22,5 g Natriumdioctylsulfosuccinat,
14,8 g Talkum AT 1,
59,4 g Bariumsulfat (Blanc Fixe Mikron),
17,3 g KPK® (Bayer AG, kationische Polyurethandispersion) und
140,0 g DMF
wurden mit Hilfe eines schnelldrehenden Rührers (Dissolver) zu einer homogenen Dispersion verarbeitet. Nach Entgasen im Vakuum wurde diese Gießlösung mit Hilfe eines Rakelmessers in einer Schichtdicke von 150 µm auf ein 200 µm dickes Polypropylenvlies (Type FO 2430 der Fa. Freudenberg) beschichtet und 3 Min. in Wasser bei 45° koaguliert. Die dabei entstandene auf der Trägerfolie ruhende Polymermatrix wurde mit Warmluft getrocknet.

b) Aufbringen der porenfreien PU-Membran: (Herstellen der erfindungsgemäßen Verbundmembran)

Die nach a) erhaltene poröse Membranmatrix wurde mit folgendem Polyurethan beschichtet:
100,0 g Polyhexandioladipat (mittleres Molekulargewicht etwa 850),
57,5 g Isophorondiisocyanat und
23,7 g Isophorondiamin
ließ man in bekannter Weise miteinander reagieren. Eine 30 %ige Lösung (Gewicht/Volumen) dieses Polyurethans in einem Gemisch von Toluol und Isopropanol (1:1) wurde durch eine Drucknutsche filtriert und so lange stehen gelassen, bis sie blasenfrei war. Diese Polyurethangießlösung wurde mit einem Naßauftrag von 100 µm auf die in a) beschriebene makroporöse Trägermembran aufgetragen. Mit Hilfe von Warmluft wurde das Lösungsmittel entfernt; dabei wurde die in Fig. 1 und 2 charakterisierte Verbundmembran Nr. 2 erhalten.

Durch Beschichten einer Polyamid-Mikrofiltrations-(MF)-membran (Fa. Pall, 0,2 µm) mit derselben Polymergießlösung nach b) unter denselben Herstellparametern wurde die in Fig. 1 und 2 charakterisierte

Membran Nr. 3 erhalten (zum Vergleich).

Beispiel 2 (zum Vergleich)

Herstellen der trägerfreien Polyurethan-Pervaporationsmembran

Die in Beispiel 1b) beschriebene Polymergießlösung wurde in 100 µm Schichtdicke auf eine transparente Polyethylenterephthalatfolie (PET-Folie) beschichtet. Das Lö sungsmittel wurde durch Abdampfen mit Warmluft entfernt; dabei wurde der auf der PET-Folie haftende Membranfilm erhalten. Durch vorsichtiges Abziehen von der PET-Folie wurde die in Fig. 1 bzw. 2 charakterisierte Membran Nr. 1 erhalten.

Beispiel 3

Herstellen einer Verbundmembran mit einer porenfreien Acrylat-Polyurethan-Blend-Trennschicht

Zu einer Polyurethan-Gießlösung aus

25,0 g Polyurethan (chem. Struktur wie im Beispiel 1b),
37,5 g Toluol und
37,5 g Isopropanol wurden
3,75 g Trimethylolpropantriacrylat (Handelsprodukt Fa. Röhm) und
0,18 g 1-Hydroxycyclohexylphenylketon (Irgacure 184®, Handelsprodukt Fa. Ciba-Geigy) als Photoinitiator gegeben.

Die Mischung wurde durch Rühren homogenisiert und zum Entgasen stehen gelassen. Diese Gießlösung wurde dann auf die im Beispiel 1a) beschriebene Polymerblendmembran in einer Schichtdicke von 150 µm aufgebracht und anschließend das Lösungsmittel abgedampft. Die dabei entstandene porenfreie Acrylat-Polyurethan-Blendschicht wurde mit Hilfe von UV-Licht vernetzt.

Belichtungsbedingungen:

Belichtungsgerät: Hanovia
Strahlenquelle: Mitteldruck Quecksilberdampflampe
Lampenleistung: 80 W/cm
Abstand von Probe zu Lampe: 11 cm
Bandgeschwindigkeit: 10 m/min

Die Trennwirkung und Durchflußcharakteristik dieser Membran entsprach bei der Toluol/Cyclohexan-Trennung der in Beispiel 1 (Fig. 1) beschriebenen Membran. Jedoch konnten bei hohen Temperaturen, etwa im Bereich von 90 °C, verbesserte Membranstabilitäten beobachtet werden.

Beispiel 4

Toluol/Cyclohexan-Separation

Mit Hilfe eines Pervaporator-Moduls, wie er z.B. in DE-OS 34 41 190 beschrieben ist, wurden die in den Beispielen 1 und 2 beschriebenen Membran unter gleichen Bedingungen durch Überströmen mit verschieden zusammengesetzten Feed-Lösungen getestet. Die Versuchsbedingungen und die Versuchsergebnisse sind in Fig. 1 und 2 dargestellt.

Auffallend ist die Selektivitätssteigerung bei erfindungsgemäßer Verwendung der makroporösen Polymerblendmembran als Verbundkomponente im Vergleich mit der Membran Nr. 1. Während die erfindungsgemäße Verbundmembran mehrere Tage bei 50 °C voll funktionsfähig blieb, löste sich die Polyurethanmembran Nr. 1 bei diesen Bedingungen nach einigen Stunden auf.

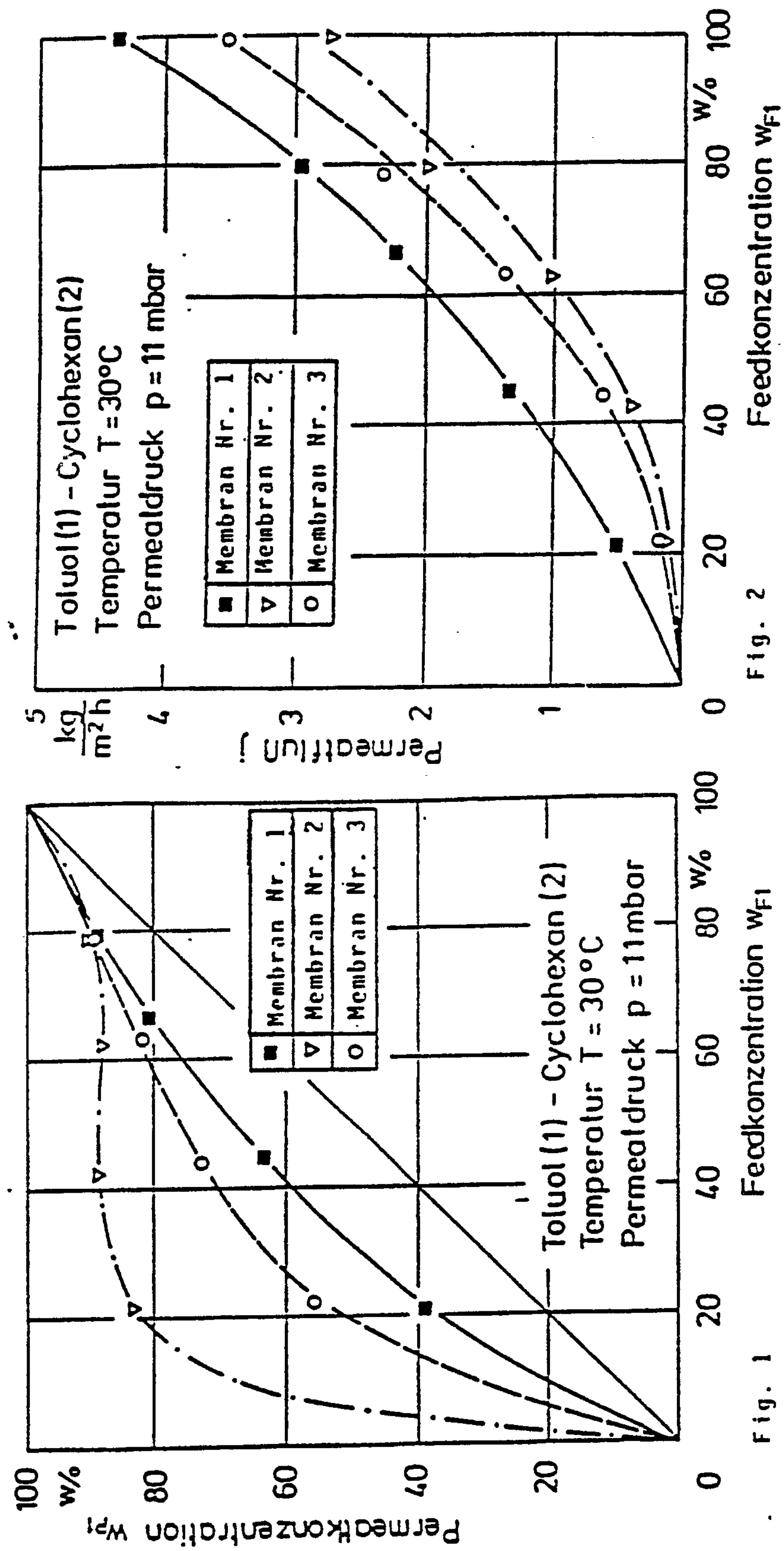


Fig. 1 und 2: Trennwirkung und Durchflußcharakteristik der Membranen nach Beispiel 1 bis 3 (Membran Nr. 1: Porenfreie Polyurethan(PU)membran; Membran Nr. 2: erfindungsgemäße Verbundmembran; Membran Nr. 3: Porenfreie PU-Membran im Verbund mit einer Polyamidmembran).

Erläuterung zu Fig. 1 und 2:

Auf der Abszisse ist jeweils die Zusammensetzung des zu trennenden Stoffgemisches (Feed) mit steigendem Toluolgehalt angegeben. Auf die Ordinate ist in Fig. 1 die Permeatkonzentration mit steigendem Toluolgehalt und in Fig. 2 der entsprechende Permeatfluß angegeben. Die erfindungsgemäße Verbundmembran Nr. 2 zeigt insbesondere im Bereich niedriger Toluolkonzentrationen eine unerwartete Selektivitätserhöhung (Vergrößerung des Trennfaktors α). Die makroporöse, Füllstoffe enthaltende Membran (i) aus mindesens 2 unverträglichen Polymeren trägt also zur selektierenden Wirkung bei, obwohl sie wegen der makroporösen Struktur dem Feed keinen Widerstand entgegensetzt und damit keine nach der Vorstellung des Löslichkeits-/Diffusionsmodells entsprechende Trennwirkung entfaltet. Zusätzlich ist die erfindungsgemäße Verbundmembran insgesamt mechanisch und chemisch auch bei höheren Temperaturen stabiler.

Beispiel 5

Abtrennung von Chlorbenzol aus einem Abwasser

Die zu reinigende Feed-Lösung war ein Abwasser, das 10 % Ethanol und 150 ppm Chlorbenzol enthielt. Zum Einsatz kann die Verbundmembran Nr. 2 aus Beispiel 1. Die Feed-Lösung wurde statisch (ohne Überströmen) an der Membran gehalten (T = 30° C; Permatdruck p = 11 mbar).

Nach einer Versuchsdauer von 4 Stunden war der Gehalt an Chlorbenzol auf 0,02 ppm in der Feed-Lösung reduziert worden.

Beispiel 6

Trennung von Benzol/Cyclohexan

Zum Einsatz kam die Verbundmembran Nr. 2 aus Beispiel 1. Zusammensetzung der Feed-Lösung: 55 % Benzol, 45 % Cyclohexan.

Die Versuchsdurchführung erfolgte wie in Beispiel 4. Es wurde ein Durchfluß von 0,6 $1/m^2$ .h ermittelt. Im Permeat konnten nur Spuren (< 0,5 % Cyclohexan) festgestellt werden.

Beispiel 7

a) Herstellung einer makroporösen, Füllstoff enthaltenden Polymerblendmembran:

21,6 g einer 17 proz. Dralon U®/DMF-Lösung,
62,5 g einer 20 proz. KBH®-Polyurethan/DMF-Lösung,
86,6 g einer 25 proz. Mowilith®/DMF-Lösung,
1,5 g Dodecylbenzolsulfonsäure-Natriumsalz,
74,2 g Talkum AT 1 und
80,0 g Dimethylformamid (DMF)
wurden wie in Beispiel 1 zu einer makroporösen Trägermembran verarbeitet.

b) Aufbringen der porenfreien PU-Membran (Herstellung der erfindungsgemäßen Verbundmembran)

Die nach a) erhaltene poröse Membranmatrix wurde mit folgendem Polyurethan beschichtet:
100,0 g Polybutandioladipat,
10,0 g Butandiol und
38,7 g Diphenylmethandiisocyanat
ließ man in bekannter Weise miteinander reagieren. Eine 30 gew.-%ige Lösung dieses Polyurethans in einem Gemisch von Dimethylformamid/Butanol (3:2) wurde in Analogie zu Beispiel 1b) hergestellt und auf die in a) beschriebenen Trägermembran beschichtet.

Zur Trennung durch Pervaporation wurde ein Benzingemisch eingesetzt, das nach gaschromatographischer Analyse 55 Komponenten mit einer Konzentration von mehr als 1 Prozent enthielt.

Die analytische Untersuchung des Permeats und des Retentats auf Aromaten zeigte nach einer eintägigen Pervaporation folgende Ergebnisse:

| | Retentat | Permeat |
|---|---|---|
| Benzol | 4 % | 10 % |
| Toluol | 7 % | 17 % |
| o-Xylol | 6 % | 8 % |
| p/m-Xylol | 18 % | 24 % |

Wie die Ergebnisse zeigen, führte die Pervaporation zu einer deutlichen Abreicherung an Benzol und Toluol.

Anhang:

Chemische Strukturen der bevorzugt eingesetzten Polymeren

Polyurethan (KBH®, Bayer AG)

Thermoplastisches Polyaddukt, welches durch Umsetzung von 75 Teilen eines Polyesters aus Adipinsäure, Ethylenglykol und 1,4-Butandiol (MG = 2000),
25 Teilen eines Polyesters aus Adipinsäure und 1,4-Butandiol (MG = 2250),
25 Teilen 1,4-Butandiol und
85 Teilen Diphenylmethan-4,4′-diisocyanat
erhalten wurde.

Dralon T® (Bayer AG)

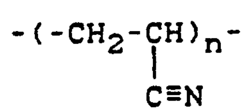

$M_n$ : 75 000

Dralon U® (Bayer AG)

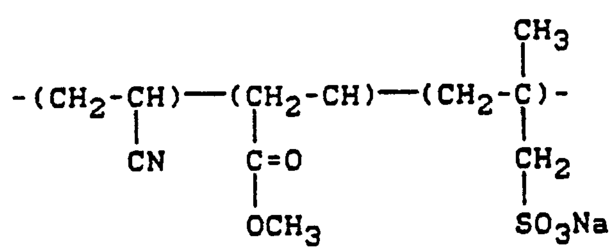

$M_n$ : 48 000

91,5 Gew.-% 5,0 Gew.-% 3,5 Gew.-%

Dralon A® (Bayer AG)

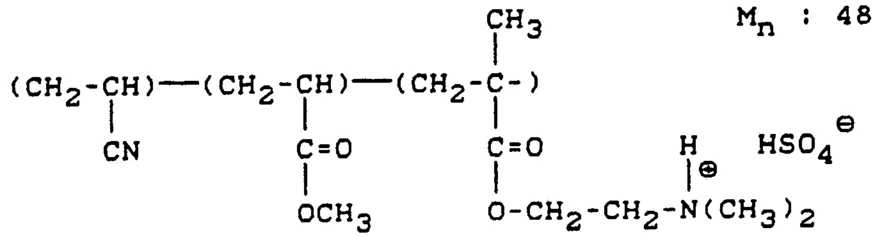

$M_n$ : 48 000

91,4 Gew.-% 4,9 Gew.-% 3,7 Gew.-%

Mowilith 50® (Polyvinylacetat, Hoechst AG)

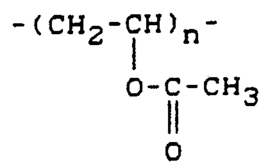

$M_n$ = 73 000

Kationische Polyurethandispersion (KPK®, Bayer AG)

Die Polyurethandispersion dient als Koagulationshilfsmittel und ist eine kationische, emulgatorfreie Dispersion eines Umsetzungsproduktes aus
200 Teilen eines Polyesters aus Adipinsäure, Phthalsäure und Ethylenglykol (MG = 1700),
50 Teilen Toluylendiisocyanat,
20 Teilen N-Methyldiethanolamin und
6 Teilen p-Xylylendichlorid.

**Ansprüche**

1. Verbundmembranen, bestehend aus
i) einer makroporösen, mindestens einen Füllstoff enthaltenden Membran aus mindestens zwei unverträglichen Polymeren, wobei der Füllstoff oder ein Gemisch mehrerer von ihnen 30 - 85 % des Gesamtgewichts von Füllstoff(en) und unverträglichen Polymeren ausmacht, und
ii) einer auf i) angebrachten porenfreien Polyurethan(PU)-membran.

2. Verbundmembranen nach Anspruch 1, dadurch gekennzeichnet, daß zwei oder drei unverträgliche Polymere aus der Klasse Polyurethane, Polyacrylnitril, Polyvinylacetat, Polystyrol, Polysulfon, Polyvinylidenfluorid, Polyamid, Polyhydantoin und Ethylenvinylacetat-copolymere mit mindestens 50 Gew.-% Vinylacetat für i) eingesetzt werden.

3. Verbundmembranen nach Anspruch 1, dadurch gekennzeichnet, daß ein oder mehrere Füllstoffe aus der Gruppe Talkum, mikrokristalline Cellulose, Zeolithe, Bentonite, $SiO_2$, $TiO_2$ und $BaSO_4$ eingesetzt werden.

4. Verbundmembranen nach Anspruch 1, dadurch gekennzeichnet, daß sie zusätzlich ein Stützmaterial enthalten, auf dem i) und dann ii) angebracht sind.

5. Verbundmembran nach Anspruch 1, dadurch gekennzeichnet, daß die porenfreie Membran ii) ein vernetztes Acrylat-Polyurethan-Blend ist.

6. Verbundmembranen nach Anspruch 5, dadurch gekennzeichnet, daß als Acrylat ein oder mehrere Ester der Acryl- oder Methacrylsäure mit aliphatischen, cycloaliphatischen oder araliphatischen Diolen

und/oder Polyolen mit drei oder mehr OH-Gruppen, wobei die Diole 4-12 C-Atome haben, wobei bevorzugt Butandiol-1,4-acrylat, Butandiol-bis-methacrylat, Trimethylolpropan-trisacrylat, Trimethylolpropantrismethylacrylat, Pentaerithrit-tetraacrylat, Pentaerithrit-tetramethylacrylat oder Urethanacrylat eingesetzt werden.

7. Verbundmembranen nach Anspruch 6, dadurch gekennzeichnet, daß als Acrylat Trimethylolpropantrisacrylat eingesetzt wird.

8. Verfahren zur Herstellung von Verbundmembranen, dadurch gekennzeichnet, daß man

a) in eine Lösung, enthaltend wenigstens zwei unverträgliche Polymere in Mengen, die zu einer Phasentrennung in der Lösung führen, mindestens einen unlöslichen Füllstoff eindispergiert, wobei der Füllstoff oder ein Gemisch mehrerer von ihnen 30 - 85 % des Gesamtgewichts von Füllstoff(en) und unverträglichen Polymeren ausmacht und wobei eine homogene Gießlösung entsteht,

b) diese Lösung zu Membranen in Form von Folien, Röhren, Schläuchen oder Hohlfasern verarbeitet und eine Fällungskoagulation durchführt und

c) auf die hierbei erhaltene makroporöse Membran eine porenfreie Polyurethanmembran aufbringt.

9. Verfahren nach Anspruch 8, dadurch gekennzeichnet, daß bei der Herstellung der Verbundmembranen in Form von Folien als Trägersubstrat für das Vergießen der unverträgliche Polymere und Füllstoffe enthaltenden Lösung ein Stützmaterial benutzt wird, welches an der Verbundmembran verbleibt.

10. Verwendung von Verbundmembranen nach Anspruch 1 zur Abtrennung von Benzol, das ein- bis dreifach durch Chlor, Brom, Hydroxyl oder $C_1$-$C_4$-Alkyl substituiert sein kann, aus aliphatischen und/oder cycloaliphatischen Kohlenwasserstoffen, Alkoholen, Ethern, Ketonen und/oder Carbonsäureestern oder aus Abwasser.